Europäisches Patentamt

European Patent Office

Office européen des brevets

(11) Numéro de publication: **0 493 275 B1**

## FASCICULE DE BREVET EUROPEEN

(45) Date de publication de fascicule du brevet: **07.12.94** (51) Int. Cl.5: **B01J 27/192**, B01J 37/00

(21) Numéro de dépôt: **91420454.0**

(22) Date de dépôt: **16.12.91**

(54) **Procédé de préparation de catalyseurs enrobés à base de molybdates de bismuth et de fer dopés par du phosphore et du potassium.**

(30) Priorité: **20.12.90 FR 9016389**

(43) Date de publication de la demande:
**01.07.92 Bulletin 92/27**

(45) Mention de la délivrance du brevet:
**07.12.94 Bulletin 94/49**

(84) Etats contractants désignés:
**BE DE ES FR GB IT NL**

(56) Documents cités:
**FR-A- 2 332 803**
**FR-A- 2 481 146**
**US-A- 4 332 971**
**US-A- 4 521 618**

(73) Titulaire: **RHONE-POULENC CHIMIE**
**25, Ouai Paul Doumer**
**F-92408 Courbevoie Cédex (FR)**

(72) Inventeur: **Legendre, Olivier**
**24, Avenue de l'Orée du Bois**
**F-95220 Herblay (FR)**
Inventeur: **Jaeger, Philippe**
**13,rue Gérard Toutain**
**F-95170 Deuil-la-Barre (FR)**

(74) Mandataire: **Dubruc, Philippe et al**
**RHONE-POULENC CHIMIE,**
**Direction de la Propriété Industrielle,**
**25, Ouai Paul Doumer**
**F-92408 Courbevoie Cédex (FR)**

## Description

La présente invention a pour objet un procédé de préparation de catalyseurs enrobés à base de molybdates de bismuth et de fer dopés par du phosphore et du potassium.

L'invention concerne plus particulièrement un procédé de préparation de tels catalyseurs enrobés comprenant la préparation d'une composition intermédiaire catalytiquement active, sa calcination suivie de son broyage, l'enrobage de particules d'un support solide et inerte dont la surface externe est rugueuse par ladite composition broyée ou un mélange la renfermant suivi de la calcination des particules ainsi enrobées.

Dans le brevet français FR-B-2 047 199 sont envisagés des catalyseurs d'oxydation répondant à la formule générale :

$$Ni_a \ Co_b \ Fe_c \ Bi_d \ L_e \ M_h \ Mo_f \ O_g$$

dans laquelle :
- L peut notamment représenter le phosphore
- M peut notamment représenter le potassium
- a et b sont compris entre 0 et 15 et la somme (a + b) entre 2 et 15
- c est compris entre 0,5 et 7
- d est compris entre 0,1 et 4
- e est compris entre 0 et 4
- f vaut 12
- g est compris entre 35 et 85
- h est compris entre 0,01 et 0,5

Ces catalyseurs sont préparés en formant une suspension en milieu aqueux à partir de divers précurseurs des constituants élémentaires du catalyseur, en ajoutant un support (tel un gel de silice) à ladite suspension semblable à une pâte, en chauffant à siccité pour obtenir un gâteau qui est alors traité à température élevée en présence d'air. Les catalyseurs sont employés sous forme de grains ou de comprimés.

Ces catalyseurs, massiques et dilués sont productifs mais ils peuvent induire des difficultés lors de la conduite d'un procédé d'oxydation à l'échelle industrielle. En effet, en lit fixe, des températures ponctuelles élevées, qui peuvent être observées, sont à l'origine d'un emballement indésirable de la réaction.

Le brevet français FR-B-2 202 729 montre qu'il est avantageux de mettre en oeuvre des catalyseurs d'oxydation du propylène en acroléine préparés par enrobage, c'est-à-dire constitués d'une couche catalytiquement active de composition analogue mais déposée à la surface externe d'un support inerte d'au moins 20 micromètres de diamètre au lieu de la diluer par un support introduit avec les sels métalliques. Il est alors possible de mieux contrôler le dégagement de chaleur de la réaction qui s'effectue dans des lits fixes.

Néanmoins, ce mode particulier de mise en forme du catalyseur nécessite qu'une part importante de celui-ci soit réservée au support inerte. La part réservée à la phase active par rapport aux catalyseurs précédents simplement dilués est diminuée ce qui entraîne une baisse très pénalisante de l'activité des catalyseurs.

Ceci peut se traduire industriellement par l'obligation soit de recourir à des réacteurs plus gros pour conserver la capacité de production et des conditions opératoires identiques soit d'augmenter la température de réaction pour conserver la capacité de production et la taille du réacteur. Dans le premier cas l'inconvénient majeur est d'ordre économique. Dans le second cas, deux inconvénients se superposent : la sélectivité en acroléine sera plus faible et l'activité du catalyseur diminuera plus rapidement au cours du temps.

Le brevet américain US-A-4 298 763 préconise, pour l'oxydation du propylène en acroléine, une composition catalytique calcinée (phase active), répondant à la formule générale :

$$Mo_{12} \ Bi_{0,1-4} \ Fe_{0,5-6} M2_b \ M3_c \ M4_d \ M5_e \ O_x$$

dans laquelle :
- M2 est du nickel et/ou du cobalt
  b vaut de 2 à 12
- M3 peut être notamment K
  c vaut de 0,01 à 0,1 et de préférence, de 0,03 à 0,09
- M4 est P
  d vaut de 0 à 1 et de préférence, de 0,01 à 0,2

- M5 peut être In et/ou Na

   e vaut de 0 à 0,5 et de préférence, de 0,01 à 0,2
- x est le nombre d'atomes d'oxygène requis pour saturer les valences des autres constituants.

Cette phase active est déposée en couche d'une épaisseur de 150 à 1500 $\mu$m sur un support qui est aussi un noyau central d'un diamètre supérieur à 100 $\mu$m et d'une surface inférieure à 15 m$^2$/g.

Le dépôt de la couche de matériau catalytique, calciné et pulvérulent, la dimension des particules étant comprise entre 0,1 et 300 $\mu$m, est réalisé en milieu humide, les particules de support étant vigoureusement agitées, des conditions opératoires contrôlées étant par ailleurs précisées.

La couche enrobant le noyau central de support représente au moins 50 % du poids du support soit au moins 33 % en poids du catalyseur fini et, au maximum 250 % du poids du support, soit au plus 71,4 % en poids du catalyseur fini.

Avant son utilisation dans l'oxydation des oléfines, le catalyseur enrobé est séché et, le cas échéant, calciné à une température de 400 à 700° C.

Il ressort de l'enseignement même de ce document que l'ensemble des constituants de la composition catalytique enrobant le support en cause est présent antérieurement à la calcination et au broyage de la dite composition.

L'exemple 1 divulgue une composition renfermant pour 12 atomes de molybdène 0,06 atome de Phosphore et 0,06 atome de Potassium, les deux éléments étant introduits dans les solutions aqueuses sous forme de KOH et d'acide phosphorique avant la précipitation, et l'atomisation de l'ensemble de la suspension. Le précurseur atomisé est ensuite remalaxé avec une autre solution aqueuse de potasse, avant extrusion puis séchage et calcination.

Les dopants sont donc introduits dans le précurseur de la phase active, avant que cette phase active ne se forme lors de la calcination. Ce mode d'introduction présente les inconvénients majeurs suivants :

- une plus grande complexité puisqu'il faut introduire de la potasse de deux façons différentes, ce qui oblige à deux pesées et donc introduit deux risques d'erreurs
- une perte importante de dopants phosphore à l'intérieur des grains de précurseurs alors que le rôle favorable des dopants s'exerce en surface des grains de phase active, là où se produisent les réactions catalytiques.
- un mauvais contrôle de la répartition microscopique du potassium ajouté ultérieurement à l'atomisation, par malaxage. Un tel précurseur, juste séché par atomisation, présente généralement des sites d'absorption des cations de force suffisante pour gêner la répartition homogène de tout le Potassium sur tous les grains de solides. Il s'ensuit un mauvais contrôle du rapport atomique Phosphore sur Potassium à l'échelle des grains, rapport atomique dont la valeur affecte significativement l'activité du catalyseur fini.

Le brevet américain US-A-4 621 072 concerne notamment un procédé de préparation de catalyseurs enrobés résistants à l'abrasion, comportant un support inerte de surface rugueuse et de dimension de particule de 0,5 à 6 mm et une couche d'un matériau catalytiquement actif enrobant le support et ancrée dans le dit support.

Ce document explique de manière détaillée les difficultées rencontrées dans la préparation de catalyseurs enrobés pour obtenir une couche enrobante aux propriétés mécaniques suffisantes pour un usage des catalyseurs à l'échelle industrielle dans des réacteurs à lit fixe. Ce document propose donc diverses mesures pour palier ces inconvénients et préconise entr'autre d'utiliser une suspension d'un précurseur du matériau catalytiquement actif contenant également un liant et, le cas échéant, un agent porogène. Là encore, le précurseur renferme tous les constituants nécessaires à la formation d'un matériau catalytiquement actif par un traitement thermique spécifique ultérieur de calcination.

Ajoutons qu'un soin particulier est apporté au procédé d'enrobage pour permettre d'obtenir une bonne résistance mécanique malgré un taux massique de phase active dans le catalyseur fini important.

Là encore le mode d'introduction éventuelle des dopants potassium et/ou phosphore présente les inconvénients déjà évoques.

En outre dans ce procédé comme dans l'ensemble de ceux déjà évoqués, les sels solubles convenables utilisés dans la préparation de la phase catalytiquement active sont les nitrates métalliques, le molybdate d'ammonium, le nitrate de potassium et l'acide phosphorique. Il se formera donc lors de cette préparation du nitrate d'ammonium qui se décomposera thermiquement à environ 230° C lors de la calcination ultérieure.

Il est clair que cette décomposition thermique du nitrate d'ammonium pose de sérieux problèmes pratiques. En effet, l'homme de l'art sait bien que le nitrate d'ammonium est un composé explosif et, à ce titre, est d'une manipulation dangereuse à l'échelle industrielle.

3

On connaît par ailleurs le brevet français FR-B-2 481 146 qui décrit un procédé pour la préparation de catalyseurs à base d'oxydes de molybdène et/ou tungstène et d'oxydes d'autres métaux. Ce procédé permet d'éviter la décomposition thermique du nitrate d'ammonium. Ce procédé est caractérisé en ce que, dans une première étape, l'on ajoute à une première solution aqueuse contenant les sels d'ammonium de molybdène et de tungstène une deuxième solution contenant les sels métalliques et que, dans une seconde étape, on ajoute au mélange obtenu de l'ammoniaque jusqu'à obtention d'un pH supérieur au pH de la première solution aqueuse de départ, puis en ce que l'on filtre la suspension obtenue pour obtenir le gâteau qui sera ensuite calciné pour faire la phase active. Ce procédé permet bien de séparer le nitrate d'ammonium d'une part, soluble dans les eaux-mères, et le précurseur de la phase active d'autre part, sous la forme d'un gâteau de filtration. Il peut être appliqué aux formulations à base de molybdate de bismuth et de fer décrites dans les brevets précédents à ceci près que, l'homme de l'art sachant bien que le nitrate de potassium et l'acide phosphorique sont solubles dans les conditions utilisées, ces éléments seront entraînés dans les eaux-mères. Il n'est donc pas possible d'introduire ainsi de façon contrôlée une quantité déterminée de phosphore et de potassium dans la phase active.

On constate donc que subsiste, malgré un art antérieur riche en enseignements, le besoin d'un procédé de préparation de compositions catalytiques enrobées à base notamment de molybdate de bismuth et de fer et le cas échéant, d'autres éléments, contenant du phosphore et du potassium, éliminant les risques précités, relativement simple à mettre en oeuvre et permettant d'assurer une répartition convenable des dopants dans la couche catalytiquement active, un contrôle précis de la composition catalytique finale et une reproductibilité satisfaisante. Il serait également hautement souhaitable de disposer d'un tel procédé pour préparer des catalyseurs enrobés de résistance mécanique satisfaisante et dans lesquels les dopants, phosphore et potassium, puissent être introduits en quantité la plus faible possible.

La présente invention a donc pour objet un procédé de préparation de catalyseurs enrobés du type molybdates de bismuth et de fer, dopés notamment par du phosphore et du potassium, comprenant la préparation d'une composition intermédiaire catalytiquement active, sa calcination suivie de son broyage, l'enrobage de particules d'un support solide et inerte dont la surface externe est rugueuse par ladite composition broyée ou un mélange la renfermant suivi de la calcination des particules ainsi enrobées, caractérisé en ce que

a) on prépare une composition intermédiaire catalytiquement active exempte de potassium et de phosphore, et en ce que

b) l'introduction du potassium et du phosphore est réalisée lors de l'étape d'enrobage.

Le procédé selon la présente invention comprend donc la préparation d'une composition intermédiaire catalytiquement active exempte de potassium et de phosphore.

La nature précise de la composition intermédiaire en cause (phase active non dopée ou intermédiaire) n'est pas déterminante dans le cadre de ce procédé, dès lors qu'elle contient les éléments chimiques classiquement utilisés pour la fabrication de catalyseurs performants d'oxydation du propylène en acroléine. Elle est essentiellement constituée de molybdate de bismuth et de fer et peut être représentée par la formule suivante :

$$A_a Mo_b W_c Fe_d Bi_e F_f G_g O_x$$

dans laquelle :
- A représente au moins un métal choisi dans le groupe constitué par le cobalt, le nickel, le manganèse, le zinc, le magnésium, le plomb, le chrome, le vanadium, le cérium et le lanthane.
- F représente au moins un élément dans le groupe constitué par l'arsenic, l'indium, l'antimoine, l'étain, le tellure, le sélénium, le silicium, le soufre et le bore
- G représente au moins un élément dans le groupe constitué par les métaux alcalino-terreux, le niobium et le thallium.
- a représente la somme des indices affectés à chacun des métaux A ; il est compris entre 0 et 12
- b et c sont chacun compris entre 0 et 12, leur somme étant égale à 12
- d est compris entre 0,2 et 6, préférentiellement entre 0,5 et 3
- e est compris entre 0,2 et 6, préférentiellement entre 0,5 et 3
- f représente la somme des indices affectés à chacun des éléments F ; il est compris entre 0 et 4, préférentiellement entre 0,5 et 2
- g représente la somme des indices affectés à chacun des éléments G ; il est compris entre 0 et 4, préférentiellement entre 0,5 et 2.

Cette composition intermédiaire (phase catalytique non dopée) est préparée selon des méthodes connues en elles-mêmes et en particulier selon les méthodes de précipitation décrites dans les brevets

4

français FR-B-2 481 146 et 2 491 778. La méthode décrite dans le premier brevet cité peut être résumée comme suit :

Dans une première étape, on prépare une solution des sels solubles, nitrates ou chlorures, des métaux du groupe A et du fer et du bismuth dans une première solution aqueuse, acidifiée par de l'acide nitrique pour éviter l'hydrolyse du bismuth. Le pH de cette première solution est compris entre 1 et 2. Dans une seconde étape, on introduit cette première solution dans une solution d'heptamolybdate d'ammonium, éventuellement de paratungstate d'ammonium et de composés solubles des éléments F et G, sous une vigoureuse agitation. Il se produit une première précipitation. Dans une troisième étape, on ajoute de l'ammoniaque toujours sous agitation, jusqu'à faire remonter le pH entre environ 6 et environ 9 et ainsi compléter la précipitation. Il est possible d'ajouter des dopants F et G à ce moment sous la forme de composés insolubles.

La solution d'ammoniaque contient entre 50 et environ 250 g d'ammoniac et elle est ajoutée à une vitesse comprise entre environ 20 et environ 200 g d'ammoniac par heure et par litre de mélange. Il est préférable de chauffer ensuite la suspension entre 30 et 100 °C pendant environ une heure pour parfaire la précipitation des espèces. Puis, la suspension est filtrée.

Le gâteau de filtration est ensuite étalé sur une épaisseur inférieure à 6 cm, puis placé dans des fours. La calcination s'effectue en montant progressivement la température à raison de 100 à 200 °C par heure. La température est ensuite maintenue à une valeur stable entre 400 et 460 °C pendant 6 heures puis le refroidissement est effectué en quelques heures.

La composition intermédiaire, ainsi obtenue, est alors broyée par des moyens connus pour que sa granulométrie n'excède pas 400 micromètres.

Le procédé selon la présente invention comprend l'enrobage de particules d'un support solide.

Les supports qui peuvent être employés dans le cadre du procédé selon l'invention se présentent sous la forme de billes pleines de diamètre compris entre 0,5 et 6 mm, valeur précise que l'homme de l'art choisira en fonction de la perte de charge admissible dans le réacteur d'oxydation. La nature chimique de ce support n'est pas critique, pourvu qu'il soit inerte chimiquement vis-à-vis des réactifs. On emploiera avantageusement de la silice, de l'alumine, de la silice-alumine, de l'argile frittée, du carborandun, de la magnésie ou du silicate de magnésium.

Il est souhaitable que le support présente une ruguosité de surface qui peut être définie par la hauteur des aspérités rapportée au diamètre moyen de la bille. Ce rapport est de préférence compris ente 0,1 et 0,2.

L'enrobage est l'opération par laquelle on entoure progressivement des billes de support d'une couche externe de phase active. Cette opération est réalisée de manière en soi connue par introduction desdites billes dans un drageoir tournant muni de moyens pour introduire des particules de phase active broyée comme indiqué ci-avant et de moyens pour introduire une solution aqueuse d'agent collant. Selon une caractéristique essentielle du présent procédé l'introduction du potassium et du phosphore est réalisée lors de l'étape d'enrobage.

Selon un mode de réalisation on introduit dans le drageoir au moins un composé du potassium et au moins un composé du phosphore sous forme de poudre finement broyée, simultanément avec la composition catalytique intermédiaire et une solution d'un agent collant.

Selon un mode de réalisation avantageux, cette introduction est réalisée dans le drageoir en cause par introduction d'une solution aqueuse d'au moins un composé de potassium et d'au moins un composé du phosphore. De préférence, la solubilité dans l'eau du ou des composés du phosphore et celle du ou des composés du potassium, mesurée à 25 °C, supérieure à 10 g/l.

Les composés du potassium et du phosphore seront choisis parmi ceux qui sont inertes vis-à-vis des autres composants de la solution d'enrobage, notamment l'agent collant et le cas échéant, le porogène. Ces composés doivent en outre être décomposables par la chaleur lors de la calcination ultérieure et ne pas détruire les agents collants et les porogènes. En pratique, l'addition des composés choisis du phosphore et du potassium doit conduire la solution aqueuse à une valeur de pH comprise entre 3 et 11.

La potasse est un composé convenable du potassium ; les phosphates minéraux ou organiques, l'acide phosphorique sont des exemples de composés convenables du phosphore.

Selon une variante préférée de la présente invention les deux éléments sont introduits simultanément par recours à un composé commun tels le dihydrogénophosphate de potassium et le monohydrogénophosphate de potassium, le complément éventuellement nécessaire en chacun des éléments pouvant être apporté par un ajout soit de potasse, soit d'acide phosphorique. Les sels d'hétéroacides répondant à la formule générale $K_xH_{1-x}PMo_{12}O_{48}$ dans laquelle x est compris entre 0 et 3 sont aussi des exemples de composés convenables du phosphore, le cas échéant, communs au potassium.

Selon une variante avantageuse du procédé selon l'invention les composés du phosphore et du potassium sont introduits dans la solution aqueuse d'agent collant.

L'opération d'enrobage est conduite dans un drageoir tournant faisant entre 10 et 20 tours par minute dans lequel on place 80 à 160 kg de billes de support rugueuses et soigneusement dépoussiérées. On introduit alors simultanément de 30 à 50 kg/h par une goulotte, la composition catalytique intermédiaire broyée, et de 8 à 15 l/h d'une solution aqueuse d'agent collant et du(des) composé(s) de phosphore et de potassium par une pompe sous pression. Dans une variante préférée, les billes ont été préalablement humectées par une solution aqueuse d'agent collant ne contenant pas de dopant.

L'opération est poursuivie jusqu'à ce que toute la solution d'enrobage, puis toute la phase active intermédiaire soit utilisée. La rotation est maintenue quelques minutes pour bien tasser la couche de phase active sur les billes. Puis elles sont séchées par de l'air chaud, entre 80 et 150°C pendant 10 à 30 minutes et introduites dans des fours. La température de ces fours est montée linéairement en 3 à 15 heures à une valeur stable comprise entre 450 et 500°C. Puis le refroidissement s'effectue en 3 à 10 heures. Dans une variante préférée, une deuxième calcination est réalisée successivement et dans les mêmes conditions de variation de température que la première calcination. Dans une autre variante préférée, la température de calcination stable est de 480°C.

L'incorporation des dopants est pratiquement totale et reproductible : il est possible de n'introduire dans la solution aqueuse utilisée lors de l'enrobage qu'une quantité voisine (à ± 5 % près) de la quantité théoriquement requise de composé(s) du phosphore et du potassium pour obtenir la stoéchiométrie recherchée pour la composition catalytique finale.

En outre, la simplicité de mise en oeuvre du procédé selon l'invention est tout à fait remarquable. La qualité de l'incorporation constitue un avantage particulièrement remarquable lorsque l'on prépare des compositions catalytiques enrobées et particulièrement actives selon la demande de brevet européen EP-A-0 493 274 dont la phase active (couche enrobante) représente de 15 à 33 % en poids et répond à la formule générale :

$$A_a \, Mo_b \, W_c \, Bi_d \, Fe_e \, P_f \, K_g \, B_h \, C_i \, O_x$$

dans laquelle :

- A représente un atome de cobalt, nickel, manganèse, magnésium et/ou plomb, et de préférence, de cobalt et/ou de nickel
- B représente un atome d'arsenic et/ou de bore,
- C représente un atome de métal alcalin différent du potassium et/ou un atome de métal alcalino-terreux différent du magnésium,
- a représente la somme des nombres d'atomes des éléments A et est compris entre 2 et 12 inclus ; lorsque A représente le cobalt seul, a est compris entre 8 et 10 inclus ;
- b est compris entre 10 et 12 inclus
- c est compris entre 0 et 2 inclus et le total (b + c) vaut 12
- d est compris entre 0,5 et 4 inclus
- e est compris entre 0,5 et 4 inclus
- f et g sont chacun compris entre 0,005 et 0,06 inclus et, de préférence, entre 0,01 et 0,03 inclus
- h représente la somme des nombres d'atomes des éléments B et est compris entre 0 et 4 inclus
- i représente la somme des nombres d'atomes des éléments C et est compris entre 0 et 0,5 inclus et
- x est le nombre d'atomes d'oxygène requis pour saturer les valences des autres constituants.

De préférence, A représente un atome de cobalt ; le rapport f/g est avantageusement compris entre 0,3 et 3 inclus et, de préférence entre 0,5 et 1,5 inclus.

Ces compositions catalytiques à faible teneur en dopants P et K offrent simultanément et durablement une activité catalytique et une sélectivité élevées, lors de la préparation d'acroléïne par oxydation du propylène.

Les exemples ci-après illustrent la présente invention.

EXEMPLE 1 :

a) Préparation de la phase active intermédiaire.

On dissout 83,8 kg d'heptamolybdate d'ammonium dans 380 litres d'eau minéralisée dont la résistivité est supérieure à 150 000 Ohm.cm en chauffant entre 70 et 80°C. Puis on refroidit la solution entre 20 et 25°C. Le pH est compris entre 5 et 5,5.

Dans un autre réacteur, on introduit 75 litres de la même eau minéralisée que l'on porte à 80°C. Puis on introduit sous agitation 115,1 kg de nitrate de cobalt hexahydraté, 16,5 kg de nitrate de fer ferrique à 9 molécules d'eau, 2,2 litres d'acide nitrique 100 % et enfin 19,2 kg de nitrate de bismuth pentahydraté. Après dissolution complète, la température est ramenée entre 20 et 25°C.

La solution des nitrates métalliques est introduite en 30 minutes dans la solution d'heptamolybdate sous forte agitation, le pH tombe entre 1 et 1,5. Puis 75 litres d'une solution à 200 grammes par litre d'ammoniac sont ajoutés en 30 minutes dans la suspension obtenue. Le pH remonte vers 7.

La température du milieu est amenée en une heure à 60°C où elle est maintenue pendant 4 heures sous agitation, puis elle est abaissée en 30 minutes à environ 22°C.

La suspension est filtrée, puis lavée par 500 litres d'eau déminéralisée de résistivité supérieure à 150 000 Ohm.cm. Le gâteau est ensuite déposé sur des plateaux sur une épaisseur d'environ 4 à 5 cm. Les plateaux sont placés dans une étuve électrique à 120°C pendant 20 heures.

Ensuite l'étuve est amenée en 5 heures à la température de 400°C maintenue pendant 6 heures sous circulation d'air. Le refroidissement à la température ambiante s'effectue en 5 heures.

Le contenu des plateaux est introduit dans un broyeur à broches réglé pour obtenir une granulométrie inférieure à 125 micromètres. Environ 102 kg de phase active intermédiaire sont ainsi obtenus.

b) L'enrobage s'effectue dans un drageoir de 1,25 m de diamètre tournant entre 15 et 18 tours par minute et contenant au début de l'opération 125 kg de billes inertes de support, en argile frittée à haute température, soigneusement dépoussiérées. Les billes sont préalablement mouillées par une première solution de glucose à 100 g par litre pulvérisée à un débit de 10 à 12 litres par heure.

Quand toutes les billes sont mouillées, une goulotte permet d'introduire 44 kg de phase active intermédiaire en poudre en environ 50 minutes. Pendant l'introduction de la poudre, le drageoir continue à tourner et 12 litres d'une seconde solution contenant 100 g/l de glucose mais aussi 5 g/l de dihydrogénophosphate de potassium sont pulvérisés sur les billes. Une fois l'ensemble de la solution, puis l'ensemble de la phase active intermédiaire introduits, la rotation est maintenue environ quelques minutes. Enfin, une canalisation souple permet d'amener un débit de 1,3 à 1,5 m3 par heure d'air chauffé à 100°C sur les billes en rotation pour les sécher pendant 15 à 20 minutes.

Les billes ainsi enrobées et séchées sont introduites dans des étuves ventilées pour y subir une calcination finale. La température de ces étuves est progressivement amenée à 240°C en 6 heures, puis à 480°C en 8 heures. Un premier palier de 6 heures à 480°C est observé, puis un refroidissement à 150°C en 10 heures est suivi d'un retour en 10 heures à 480°C. Un second palier de 6 Heures à 480°C est suivi du refroidissement final en 10 heures jusqu'à la température ambiante.

On retire donc de l'étuve environ 169 kg de catalyseur fini dont 26 % est constitué de phase active répondant à la formule $Co_{9,6}Mo_{12}Fe_1Bi_1K_{0,028}P_{0,028}O_x$.

## EXEMPLE COMPARATIF A

On mélange dans un malaxeur 100 kg de phase active intermédiaire calcinée, préparée suivant les conditions opératoires présentées dans l'exemple 1, avec 4 litres d'une solution aqueuse contenant 270 g de dihydrogénophosphate de potassium. Le malaxage s'avère difficile car il diminue considérablement la viscosité de l'ensemble. Après environ 15 minutes, l'ensemble est ensuite séché pendant 15 heures à 120°C en étuve.

Puis 44 kg de la phase active ainsi dopée sont enrobés suivant le même protocole, à ceci près que l'on ne met pas de dihydrogénosphosphate de potassium dans la seconde solution d'enrobage.

Le séchage et la calcination sont identiques à ce qui est décrit dans l'exemple 1.

## EXEMPLE COMPARATIF B

Un autre catalyseur est préparé dans des conditions identiques à celle de l'exemple 1 sauf que l'on n'introduit pas de dihydrogénophosphate de potassium dans la solution d'enrobage.

## EXEMPLE 2 :

Incorporation et Répartition des dopants dans la couche de phase active :

Pour connaître le taux d'incorporation et la qualité de la répartition des dopants dans le catalyseur fini, il est nécessaire de doser les éléments phosphore et potassium dans l'épaisseur de la couche de phase active des catalyseurs. On procède d'abord à une séparation physique de différentes fractions de la couche de phase active par attrition mécanique.

Pour cela, on introduit 30 g de catalyseur fini dans un récipient métallique fermé de 50 ml de contenance. L'ensemble est introduit à l'intérieur d'un appareil qui produit des oscillations verticales d'environ 4 cm d'amplitude 700 fois par minute.

Au bout de 10 minutes, l'appareil est arrêté et le récipient ouvert. Les billes de catalyseurs sont séparées par tamisage des poussières de phase active décollées par attrition. Ces poussières sont collectées, pesées puis mises en solution pour doser les dopants. Le phosphore est dosé par spéctrométrie d'émission plasma et le potassium par spéectrométrie d'émission de flamme.

Ensuite, les billes sont remises dans le récipient et l'attrition est prolongée de 10 minutes supplémentaires. Une nouvelle fraction de phase active est collectée puis dosée. L'opération est reproduite deux autres fois, jusqu'à ce qu'il n'y ait pratiquement plus de phase active visible sur les catalyseurs attritionnés pratiquement réduits à leurs billes inertes centrales.

A partir de la masse de chaque collecte, il est possible d'estimer la position où se trouvait la fraction de phase active collectée en supposant que l'attrition s'est produite de façon parfaitement progressive, c'est à dire que la première collecte ne correspond qu'aux grains les plus externes et la dernière seulement aux grains les plus internes de la couche. Cette hypothèse, suffisamment proche de la réalité, permet de déterminer la fourchette de position relative (en % de l'épaisseur) où était située la fraction de phase active arrachée par l'attrition.

En raison de la rugosité des billes constituant le support, une partie des poussières collectées n'est autre que de la matière constitutive de ces billes, à savoir de l'argile. Cette proportion d'argile dans les poussières collectées est d'autant plus importante que l'attrition a été plus longue, elle est donc plus importante dans les dernières collectes. Un échantillon de ces billes inertes, en argile frittée à haute température, a donc été broyé pour pouvoir doser la quantité de potassium contenu dans l'argile du support. D'autre part, les poussières collectées sont analysées par fluorescence X pour déterminer, par la réponse de l'élément silicium relativement à des mélanges mécaniques étalonnés, la proportion d'argile qu'elles contiennent. En fin de compte, il est ainsi possible par différence de déterminer la quantité de potassium et de phosphore contenue dans chaque collecte.

Les valeurs obtenues pour le catalyseur de l'exemple 1 sont reportées dans le tableau I ci-après de façon comparative avec la valeur théorique attendue si la répartition des dopants était parfaitement homogène :

TABLEAU I

| Position (% de l'épaisseur) | Teneurs brutes | | | Teneurs sur la fraction de phase active | | Teneurs théoriques attendues | |
|---|---|---|---|---|---|---|---|
| | K ppm | P ppm | Argile % masse | K ppm | P ppm | K ppm | P ppm |
| extérieur 100 à 26 | 480 | 270 | 2 | 460 | 263 | 392 | 310 |
| 26 à 16 | 415 | 260 | 1 | 404 | 256 | 392 | 310 |
| 16 à 11 | 450 | 245 | 4 | 401 | 227 | 392 | 310 |
| intérieur 11 à 6,5 | 770 | 225 | 8 | 677 | 191 | 392 | 310 |
| argile centrale | 1110 | 410 | 100 | | | | |

Il apparaît donc que les teneurs en dopants sont globalement très proches de la valeur attendue si la répartition était bien homogène. (La différence persistante pour le phosphore s'explique peut-être par une erreur systématique de dosage, ou une légère perte dans l'argile du support). Globalement, les taux d'incorporation sont maîtrisables et proches de 100 %.

Pour ce qui est de la répartition dans l'épaisseur de la phase active, les teneurs apparaissent constantes à ± 15 % pour le potassium, si l'on excepte la valeur pour la couche la plus interne, vraisemblablement la plus perturbée par la présence d'argile. Pour le phosphore, il semble y avoir une variation de ± 30 %, apparemment plus systématique en fonction de la profondeur de la couche.

A titre de comparaison, les valeurs mesurées sur le catalyseur préparé selon l'exemple comparatif A sont indiquées dans le tableau II ci-après :

TABLEAU II

| Position (% de l'épaisseur) | Teneurs brutes | | | Teneurs sur la fraction de phase active | | Teneurs théoriques attendues | |
|---|---|---|---|---|---|---|---|
| | K ppm | P ppm | Argile % masse | K ppm | P ppm | K ppm | P ppm |
| extérieur 100 à 28 | 2260 | 920 | 2 | 2241 | 913 | 607 | 481 |
| 28 à 19 | 2315 | 890 | 1 | 2308 | 887 | 607 | 481 |
| 19 à 13 | 2190 | 890 | 1 | 2176 | 885 | 607 | 481 |
| intérieur 13 à 5,7 | 2165 | 880 | 11 | 2038 | 833 | 607 | 481 |
| argile centrale | 1110 | 410 | 100 | | | | |

Il apparaît donc clairement que les valeurs sont complètement différentes de celles qui seraient attendues si la répartition des dopants était homogène. La fraction du lot de phase active dopée examinée s'avère trop riche en dopants : environ 4 fois plus pour le potassium et 2 fois plus en phosphore. Cela est vraisemblablement du à l'inhomogénéité de répartition provoquée par le malaxage. Les taux d'incorporation des dopants n'apparaissent pas maîtrisables.

EXEMPLE 3 :

Influence des dopants sur la résistance mécanique du catalyseur fini :

La résistance mécanique des catalyseurs finis est déterminée par un test d'attribution comme suit :

100 g de catalyseur fini sont introduits dans un tambour en plexiglass® de diamètre extérieur 200 mm et de largeur 40 mm fixé sur l'axe horizontal d'un moteur tournant à 10 tours par minute. A l'intérieur du tambour sont fixés, à intervalle régulier, 6 aubes planes en plexiglass® de 45 mm de long et de 40 mm de large, inclinées à 40°C par rapport au diamètre passant par leur base de fixation.

Le sens de rotation du tambour est tel que, si l'on représente le vecteur de la vitesse tangentielle du tambour en un point de fixation de l'une quelconque des aubes, celui-ci fasse un angle de 50°C avec l'aube.

Le tambour est mis en rotation pendant 5 minutes, puis les billes sont retirées du tambour et pesées après tamisage pour séparer les fines. La masse ainsi déterminée est $m_5$. L'appareil est dépoussiéré, puis les billes sont réintroduites. Une nouvelle pesée est effectuée au bout de 10 minutes, soit une masse $m_{15}$.

Le taux d'attrition est défini comme la proportion de phase active enlevée par le dispositif d'attrition. Il est calculé par rapport au taux T de phase active de la façon suivante :

Après 5 minutes, le taux d'attrition, en %, vaut $(100-m_5) \times 100/T$

Après 15 minutes cumulée d'attrition, le taux vaut $(100-m_{15}) \times 100/T$.

Les mesures d'attrition effectuées sur différents lots de 169 kg de catalyseur préparés selon les conditions de l'exemple 1 suivant le procédé de l'invention sont reportées dans le tableau suivant, comparativement à celles effectuées sur un catalyseur préparé selon les conditions de l'exemple B, donc non dopé :

EP 0 493 275 B1

| Exemple | Dopants | Teneur T (%) | Taux d'attrition à 5 mn (%) | Taux d'attrition à 15 mn (%) |
|---|---|---|---|---|
| 1-lot 1 | K0,028P0,028 | 26 | 0,00 | 0,38 |
| 1-lot 2 | K0,028P0,028 | 26 | 0,00 | 0,08 |
| 1-lot 3 | K0,028P0,028 | 26 | 0,23 | 0,31 |
| B | K0    P0 | 26 | 0,23 | 0,46 |

Comme on considère un catalyseur comme solide à partir du moment où le résultat de ces mesures est inférieur à 1 % par minute d'attrition, il apparaît que les catalyseurs préparés selon le procédé de l'invention sont très résistants. En outre la dispersion apparente des résultats sur les différents lots n'a pas de signification physique importante, puisque les valeurs sont toutes très petites.

Il apparaît que, contrairement à ce que l'on pouvait attendre, la présence de dopants dans la solution d'enrobage ne modifie pas de manière significative la résistance mécanique du catalyseur.

EXEMPLE 4 :

Activité de la formulation préparée selon l'invention

Un échantillon de 100 ml de catalyseur préparé selon l'exemple 1 est testé dans la réaction d'oxydation du propylène en acroléïne. Le réacteur utilisé a un diamètre interne 21 mm et 50 cm de hauteur.

Le mélange réactionnel introduit dans le réacteur chauffé par un bain de sable contient en pourcentage volumique 7 % de propylène, 57 % d'air et 36 % de vapeur d'eau. Le débit de propylène est ajusté pour obtenir une charge d'environ 250 g de propylène par heure et par litre de catalyseur. La pression de sortie du réacteur est régulée à 1,8 bar absolu.

Les effluents du réacteur contiennent un mélange gazeux d'azote, d'oxygène, de vapeur d'eau, de propylène, d'acroléïne, d'acide acrylique, d'acide acétique, d'acétaldéhyde, de monoxyde et de dioxyde de carbone et d'autres impuretés en faible quantité. Des chromatographes en phase gaz permettent de déterminer les proportions en chacun de ces produits et donc de calculer les performances catalytiques, c'est-à-dire :

Le taux de transformation, noté $X_g$

$$X_g = \frac{\text{Nombre de moles de propylène disparu}}{\text{Nombre de moles de propylène à l'entrée}} \times 100$$

La sélectivité en produit i, notée $S_i$

$$S_i = \frac{\text{Nombre de moles de produit i formé}}{\text{Nombre de moles de propylène transformé}} \times 100$$

et $R_i$ qui est le rendement en produit i. Le rendement est le produit de la conversion Xg par la sélectivité en produit i : $R_i = X_g \times S_i$

Les résultats obtenus sur le catalyseur préparé selon l'exemple 1 sont reportés dans le tableau suivant :

10

EP 0 493 275 B1

| Dopants | Teneur (%) | Température de bain ° C | Xg (%) | Sacroléine (%) | Sacrylique (%) | Sco-co2 (%) | Racroléine (%) |
|---|---|---|---|---|---|---|---|
| K0,028P0,028 | 26 | 357 | 95,9 | 78,6 | 12,6 | 4,3 | 75,4 |

Ils montrent clairement que le catalyseur ainsi préparé selon le procédé selon l'invention permet d'obtenir de bonnes performances catalytiques, c'est-à-dire un rendement élevé en acroléïne à une valeur élevée de convesion du propylène.

**Revendications**

1. Procédé de préparation de catalyseurs enrobés du type molybdates de bismuth et de fer, dopés notamment par du phosphore et du potassium, comprenant la préparation d'une composition intermédiaire catalytiquement active, sa calcination suivie de son broyage, l'enrobage de particules d'un support solide et inerte dont la surface externe est rugueuse par ladite composition broyée ou un mélange la renfermant suivi de la calcination des particules ainsi enrobées, caractérisé en ce que :
   a) la composition intermédiaire catalytiquement active est exempte de potassium et de phosphore, et en ce que
   b) l'introduction du potassium et du phosphore est réalisée lors de l'étape d'enrobage.

2. Procédé de préparation de catalyseurs selon la revendication 1, caractérisé en ce que lors de l'étape d'enrobage, on introduit une solution aqueuse d'au moins un composé du potassium et d'au moins un composé du phosphore.

3. Procédé selon la revendication 2, caractérisé en ce que la solubilité dans l'eau du (ou des) composé(s) du phosphore et du (ou des) composé(s) du potassium, mesurée à 25 ° C est supérieure à 10 g/l.

4. Procédé selon la revendication 2 ou 3, caractérisé en ce que le pH de la solution aqueuse après addition du(ou des) composé(s) du phosphore et du (ou des) composés(s) du potassium est compris entre 3 et 11.

5. Procédé selon l'une quelconque des revendications 2 à 4 caractérisé en ce que la solution aqueuse renferme un agent collant.

6. Procédé selon l'une quelconque des revendications précédentes caractérisé en ce que le potassium est introduit sous forme de potasse.

7. Procédé selon l'une quelconque des revendications précédentes caractérisé en ce que le phosphore est introduit sous forme d'acide phosphorique ou d'un phosphate minéral ou organique.

8. Procédé selon l'une quelconque des revendications précédentes caractérisé en ce que tout ou partie du phosphore et du potassium est introduit sous forme de mono- ou de dihydrogénophosphate de potassium.

9. Procédé selon l'une quelconque des revendications précédentes caractérisé en ce que la quantité de composé(s) du potassium et du phosphore introduit dans la solution aqueuse correspond à ± 5 % près à la quantité théoriquement requise en potassium et en phosphore pour obtenir la stoechiométrie recherchée pour la composition catalytique finale.

10. Procédé selon l'une quelconque des revendications précédentes caractérisé en ce que la calcination des particules enrobées et réalisées à une température comprise entre 450 et 500 ° C.

**Claims**

1. Process for the preparation of coated catalysts of the bismuth and iron molybdates type, doped especially with phosphorus and with potassium, comprising the preparation of a catalytically active intermediate composition, its calcining followed by its milling, coating the particles of a solid and inert support whose outer surface is rough using the said milled composition or a mixture containing it,

11

followed by calcining of the particles thus coated, characterized in that:

a) the catalytically active intermediate composition is free from potassium and phosphorus, and in that

b) the introduction of the potassium and of the phosphorus is performed during the coating stage.

2. Process for the preparation of catalysts according to Claim 1, characterized in that during the coating stage an aqueous solution of at least one potassium compound and of at least one phosphorus compound is introduced.

3. Process according to Claim 2, characterized in that the solubility of the phosphorus compound(s) and of the potassium compound(s) in water, measured at 25° C, is higher than 10 g/l.

4. Process according to Claim 2 or 3, characterized in that the pH of the aqueous solution after addition of the phosphorus compound(s) and of the potassium compound(s) is between 3 and 11.

5. Process according to any one of Claims 2 to 4, characterized in that the aqueous solution contains an adhesive agent.

6. Process according to any one of the preceding claims, characterized in that the potassium is introduced in the form of potassium hydroxide.

7. Process according to any one of the preceding claims, characterized in that the phosphorus is introduced in the form of phosphoric acid or of an inorganic or organic phosphate.

8. Process according to any one of the preceding claims, characterized in that all or part of the phosphorus and of the potassium is introduced in the form of potassium mono- or dihydrogenphosphate.

9. Process according to any one of the preceding claims, characterized in that the quantity of the potassium and phosphorus compound(s) introduced into the aqueous solution corresponds to within ± 5 % to the quantity of potassium and phosphorus theoretically required to obtain the stoichiometry sought after for the final catalyst composition.

10. Process according to any one of the preceding claims, characterized in that the calcining of the coated particles is carried out at a temperature of between 450 and 500° C.

**Patentansprüche**

1. Verfahren zur Herstellung ummantelter Katalysatoren vom Typ der Wismut- und Eisen-Molybdate, die insbesondere mit Phosphor und Kalium dotiert sind, enthaltend die Herstellung einer katalytisch aktiven Zwischenverbindung, deren Calcinieren mit anschließendem Mahlen, das Ummanteln von Teilchen eines festen und inerten Trägers mit rauher äußerer Oberfläche mit dieser gemahlenen Verbindung oder einer diese enthaltende Mischung, und anschließendem Calcinieren der so ummantelten Teilchen, dadurch gekennzeichnet, daß

a) die katalytisch aktive Zwischenverbindung frei von Kalium und Phosphor ist und

b) das Kalium und Phosphor während des Ummantelns eingebracht werden.

2. Verfahren zur Herstellung von Katalysatoren nach Anspruch 1, dadurch gekennzeichnet, daß während des Ummantelns eine wäßrige Lösung wenigstens einer Kalium- und wenigstens einer Phosphorverbindung eingebracht wird.

3. Verfahren nach Anspruch 2, dadurch gekennzeichnet, daß die bei 25 °C gemessene Wasserlöslichkeit der Phosphor- und der Kaliumverbindung(en) größer als 10 g/l ist.

4. Verfahren nach Anspruch 2 oder 3, dadurch gekennzeichnet, daß der pH-Wert der wäßrigen Lösung nach Zugabe der Phosphor- und der Kaliumverbindung(en) zwischen 3 und 11 liegt.

**5.** Verfahren nach einen, der Ansprüche 2 bis 4, dadurch gekennzeichnet, daß die wäßrige Lösung ein Klebemittel enthält.

**6.** Verfahren nach einem der vorhergehenden Ansprüche, dadurch gekennzeichnet, daß das Kalium in Form von Kaliumhydroxid eingebracht wird.

**7.** Verfahren nach einem der vorhergehenden Ansprüche, dadurch gekennzeichnet, daß der Phosphor in Form von Phosphorsäure oder eines mineralischen oder organischen Phosphats eingebracht wird.

**8.** Verfahren nach einem der vorhergehenden Ansprüche, dadurch gekennzeichnet, daß der Phosphor und das Kalium ganz oder teilweise in Form von Kaliummono- oder Kaliumdihydrogenphosphat eingebracht wird.

**9.** Verfahren nach einem der vorhergehenden Ansprüche, dadurch gekennzeichnet, daß die Menge der in die wäßrige Lösung eingebrachten Kalium- und Phosphorverbindung(en) ± 5 % der theoretisch benötigten Menge an Kalium und Phosphor entspricht, die zum Erreichen der angestrebten Stöchiometrie der katalytischen Endverbindung erforderlich ist.

**10.** Verfahren nach einem der vorhergehenden Ansprüche, dadurch gekennzeichnet, daß das Calcinieren der ummantelten Teilchen bei einer Temperatur zwischen 450 und 500 °C durchgeführt wird.